**Europäisches Patentamt**

**European Patent Office**

**Office européen des brevets**

(19)

(11) Publication number: **0 098 700**
**B1**

(12) **EUROPEAN PATENT SPECIFICATION**

(45) Date of publication of patent specification: **06.08.86**

(21) Application number: **83303430.9**

(22) Date of filing: **14.06.83**

(51) Int. Cl.⁴: **A 61 K 35/60,** A 61 K 33/18 //
(A61K35/60, 33:18, 31:125)

(54) **Antitumor agent.**

(30) Priority: **18.06.82 JP 103742/82**

(43) Date of publication of application:
**18.01.84 Bulletin 84/03**

(45) Publication of the grant of the patent:
**06.08.86 Bulletin 86/32**

(84) Designated Contracting States:
**DE FR GB IT**

(73) Proprietor: **Mori, Tokitaka**
**1773-10 Nishi-ooizumi**
**Nerima-ku Tokyo (JP)**

(72) Inventor: **Mori, Tokitaka**
**1773-10 Nishi-ooizumi**
**Nerima-ku Tokyo (JP)**

(74) Representative: **Arthur, Bryan Edward et al**
**Withers & Rogers 4 Dyer's Buildings Holborn**
**London EC1N 2JT (GB)**

(56) References cited:

**DERWENT JAPANESE PATENTS REPORT, vol.
S, no. 31, September 14, 1971, page 4, B,
abstract no. 51334S**

**ROTE LISTE, 1961, Editio Cantor
AULENDORF/WÜRTT. (DE)- Page 420,
"Helojodan"- Page 665, "Osnol Rheuma-Rub
Organomed"- Page 838, "Schieferöl-Isapogen"**

Courier Press, Leamington Spa, England.

## Description

This invention relates to a novel antitumor agent.

Various kinds of antitumor agents including in a broad sense anti-cancer agents are known. Some of these antitumor agents have been shown to be effective to some extent on certain tumors out of various kinds of tumors. However, a decisive antitumor agent has not been yet found. Under these circumstances, studies on antitumor agents having composition different from those of conventional antitumor agents have been continued.

This invention has been accomplished as a result of study on an antitumor agent having a composition different from than those of conventional antitumor agents.

This invention resides in an antitumor agent comprising a fused mixture of camphor and liver oil containing 6.5—7.5 percent by weight of combined iodine.

Compositions containing camphor and a mineral or vegetable oil for use in treatment of ailments such as rheumatism and arthritis are known, and Japanese Patent Publication No. 46(1971)—26984 describes a fused composition containing cod liver oil, iodine and borneol which is said to be employable as an anti-hypertensive agent.

The antitumor agent of the invention shows an effect in the prevention or repression of growth of various kinds of tumors. The antitumor agent of the invention particularly shows a marked effect on prevention or repression of growth of myelogenous and lymphocytic leukemia, lymphosarcoma, malignant lymphosarcoma (Hodgkin disease), carcinoma of the colon, ovarian cancer, ileocecal tumor (cancer), lung cancer, cancer of the liver and pancreas, cancer of the breast, cancer of the digestive organ and cancer of the stomach.

The antitumor agent of the invention is usually administered orally. The dosage for an adult is usually within a range from 0.5 to 30 g/day, and preferably 1 to 20 g/day, the dosage being based on the effective component, that is, the fused composition of camphor and liver oil containing 6.5—7.5 percent by weight of combined iodine. Continuous administration of the antitumor agent of the invention shows a remarkable antitumor effect, and ordinarily does not cause side effects, provided that the dosage of the antitumor agent is within the above range.

The effective component of the antitumor agent of the invention, that is, the fused composition of camphor and liver oil containing 6.5—7.5 percent by weight of combined iodine can be prepared by, for example, the following process.

Approximately 70—90 parts by weight (preferably approximately 75—85 parts by weight) of liver oil (meeting the requirements of the Japanese Pharmacopoeia) is melted by heating to approximately 180—230°C (preferably approximately 200—220°C and more preferably around 210°C). To this are successively added approximately 6—8 parts by weight (preferably approximately 6.5—7.5 parts by weight) of iodine (meeting the requirements of the Japanese Pharmacopoeia) and approximately 7—9 parts by weight (preferably approximately 7.5—8.5 parts by weight) of camphor (melting point: 210—213°C, purity: not less than 97 percent by weight). The mixture is sufficiently stirred under heating to obtain a homogeneous and viscous oily dark brown liquid. After the mixture is made homogeneous, a small amount of a base such as sodium carbonate or sodium hydrogen carbonate is preferably added to the mixture. Subsequently, the oily liquid is cooled to room temperature to obtain the desired fused composition (viscous oily liquid) of camphor and liver oil containing 6.5—7.5 percent by weight of combined iodine.

In another process, before cooling the homogeneous and viscous oily dark brown liquid obtained in the above process to room temperature, an optional amount of creosote (meeting the requirements of Japanese Pharmacopoeia), for example, an amount of less than approximately 20 parts by weight (preferably approximately less than 10 parts by weight) is added to the oily liquid and sufficiently stirred under heating to obtain a homogeneous mixture. In this proccess, it is also preferred to add a small amount of a base such as sodium carbonate or sodium hydrogen carbonate to the stirred mixture. Subsequently, the mixture is cooled to room temperature to obtain the desired agent (viscous oily liquid) for oral administration which comprises the fused composition of camphor and liver oil containing 6.5—7.5 percent by weight of combined iodine and creosote.

Clinical examples showing the effect brought about by the administration of the antitumor agent of the present invention will be described hereinunder. The antitumor agent used in each clinical example was prepared by the following process.

To 80 parts by weight of liver oil (meeting the requirements of Japanese Pharmacopoeia) heated to about 210°C were added successively 7.0 parts by weight of iodine (meeting the requirements of the Japanese Pharmacopoeia) and 8.0 parts by weight of camphor (melting point: 210—213°C, purity: not less than 97 percent by weight) successively under vigorous stirring to obtain a homogeneous and viscous oily dark brown liquid. Subsequently, the oily liquid was allowed to stand to cool. At the time when the oily liquid had cooled to about 50°C, 5.0 parts by weight of creosote (meeting the requirements of the Japanese Pharmacopoeia) was added. The mixture was sufficiently stirred until homogeneous and then allowed to stand until cool to obtain an antitumor agent in the form of a viscous oily liquid.

The so obtained antitumor agent was a mixture of creosote and a fused composition of camphor and liver oil containing 7.0 percent by weight of combined iodine.

The antitumor agent prepared by the above process was continuously administered to the patients orally.

### Clinical Example 1

Patient: Female (69 years old)

Condition: Carcinoma of the colon was taken out by operation. After the operation, metastasis of the tumor was observed by X-ray examination. Bleeding was also observed.

Change of condition after administration of the antitumor agent: A dosage of 1.2 g was continuously administered to the patient 4 times a day. On the about 210th day after beginning the administration, bleeding ceased and the weight of the patient increased by about 1 Kg. Reduction and disappearance of the tumor were confirmed by X-ray examination.

### Clinical Example 2

Patient: Female (40 years old)

Condition: Ovarian cancer was taken out by operation. After the operation, metastasis of the tumor was observed by X-ray examination.

Change of condition after administration of the antitumor agent: A dosage of 1.2 g was continuously administered to the patient 4 times a day. On the about 180th day after beginning the administration, the condition of anemia was improved and the weight of the patient increased by about 6 Kg.

### Clinical Example 3

Patient: Male (54 years old)

Condition: Ileocecal tumor (cancer, as large as a head of a child) was taken out by operation. However, a part of the tumor could not be taken out. The X-ray examination after the operation indicated growth of the tumor (as large as a fist of an adult) and metastasis of the tumor to the rectum and the back wall of the stomach.

Change of condition after administration of the antitumor agent: A dosage of 1.5 g was continuously administered to the patient 6 times a day. On the about 150th day after beginning the administration, reduction of the ileocecal tumor (cancer) was confirmed by palpation. The X-ray examination also showed reduction of the tumor (as large as a fist of a child) and disappearance of the metastasized tumors.

### Clinical Example 4

Patient: Female (51 years old)

Condition: Cancer of the breast

Change of condition after administration of the antitumor agent: A dosage of 1.2 g was continuously administered to the patient 4 times a day. On the about 120th day after beginning the administration, the X-ray examination indicated little growth of the tumor, but showed no metastasis to the lung, bone or other internal organs.

### Clinical Example 5

Patient: Female (74 years old)

Condition: A lung cancer was taken out by operation. However, a part of the cancer could not be taken out.

Change of condition after administration of the antitumor agent: A dosage of 1.2 g was continuously administered to the patient 4 times a day. On the about 150th day after beginning the administration, the X-ray examination indicated no growth of the tumor which had not been taken out by the operation.

### Clinical Example 6

Patient: Female (32 years old)

Condition: The patient was diagnosed as suffering from malignant tumor of the lymphnode by X-ray examination and the examination of the lymph taken out from the armpit. A noticeable swelling was observed on the arm.

Change of condition after administration of the antitumor agent: A dosage of 1.2 g was continuously administered to the patient 5 times a day. On the about 90th day after beginning the administration, the weight of the patient increased and the swelling disappeared. The X-ray examination indicated that the size of the tumor had been reduced by approximately a half.

### Clinical Example 7

Patient: Male (47 years old)

Condition: Inoperable cancer of the stomach

Change of condition after administration of the antitumor agent: A dosage of 1.2 g was continuously administered to the patient 5 times a day. On the about 50th day after beginning of the administration, the pain was reduced and the appetite increased. The tumor could not be detected by palpation.

### Clinical Example 8

Patient: Female (30 years old)

Condition: The patient was diagnosed as suffering from cancer of the stomach by X-ray and gastro-camera examinations.

Change of condition after administration of the antitumor agent: A dosage of 1.2 g was continuously administered to the patient 4 times a day. On the about 90th day after beginning the administration, anemia was improved and the weight of the patient increased by about 5 Kg. It was confirmed by X-ray examination that the size of the tumor had been reduced by approximately a half.

### Clinical Example 9

Patient: Female (52 years old)

Condition: The patient was diagnosed as suffering from lymphocytic leukemia since limphoblasts such as myelocytes and metamyelocytes were detected in the blood and that diseased cells existed in the bone marrow fluid.

Change of condition after administration of the antitumor agent: A dosage of 1.2 g was continuously administered to the patient 4 times a day. On the about 30th day after beginning the administration, myelocytes and metamyelocytes were not detected on the blood. On the about 180th day after beginning the administration, the above limphoblasts were not detected in the blood, and the number of red blood cells had remarkably increased.

## Claims

1. An antitumor agent comprising a fused composition of camphor and liver oil containing 6.5—7.5 percent by weight of combined iodine.

2. The antitumor agent as claimed in claim 1 wherein the fused composition of camphor and liver oil containing 6.5—7.5 percent by weight of combined iodine is prepared by adding under stirring 6—8 parts by weight of iodine and 7—9 parts by weight of camphor to 70—90 parts by weight of liver oil kept under melted conditions by heating.

3. The antitumor agent as claimed in claim 1 wherein the fused composition of camphor and liver oil containing 6.5—7.5 percent by weight of combined iodine is prepared by adding under stirring 6.5—7.5 parts by weight of iodine and 7.5—8.5 parts by weight of camphor to 75—85 parts by weight of liver oil kept under melted conditions by heating.

4. The antitumor agent as claimed in claim 1, 2 or 3 which also contains creosote.

## Patentansprüche

1. Antitumor-Mittel, enthaltend eine verschmolzene Zusammensetzung aus Kampfer und Lebertran mit 6.5 bis 7.5 Gew. % gebundenem Jod.

2. Antitumor-Mittel nach Anspruch 1, bei welchem die verschmolzene Zusammensetzung aus Kampfer und Lebertran mit 6.5 bis 7.5 Gew. % gebundenem Jod dadurch hergestellt wird, daß 6 bis 8 Gewichtsteile Jod und 7 bis 9 Gewichtsteile Kampfer mit 70 bis 90 Gewichtsteilen Lebertran, welcher durch Heizen in geschmolzenem Zustand gehalten ist, verrührt werden.

3. Antitumor-Mittel nach Anspruch 1, bei welchem die verschmolzene Zusammensetzung aus Kampfer und Lebertran mit 6.5 bis 7.5 Gew. % gebundenem Jod dadurch hergestellt wird, daß 6.5 bis 7.5 Gewichtsteile Jod und 7.5 bis 8.5 Gewichtsteile Kampfer mit 75 bis 85 Gewichtsteilen Lebertran, welcher durch Heizen in geschmolzenem Zustand gehalten ist, verrührt werden.

4. Antitumor-Mittel nach Anspruch 1, 2 oder 3, welches zusätzlich Kreosot enthält.

## Revendications

1. Agent antitumeur constitué d'une composition de camphre et d'huile de foie contenant 6,5 à 7,5 % en poids d'iode combiné.

2. Agent antitumeur suivant la revendication 1, dans lequel la composition fondue de camphre et d'huile de foie, contenant 6,5 à 7,5 % en poids d'iode combiné, est préparée en ajoutant, tout en agitant, 6 à 8 parties en poids d'iode et 7 à 9 parties en poids de camphre à 85 à 95 parties en poids d'huile de foie, maintenue par chauffage à l'état fondu.

3. Agent antitumeur suivant la revendication 1, caractérisé en ce que la composition fondue de camphre et d'huile de foie, contenant 6,5 à 7,5 % en poids d'iode combiné est préparée en ajoutant, tout en agitant, 6,5 à 7,5 % en poids d'iode et 7,5 à 9,5 % en poids de camphre à 75 à 85 parties en poids d'huile de foie, maintenue à l'état fondu par chauffage.

4. Agent antitumeur suivant les revendications 1, 2 et 3, caractérisé en ce qu'il contient aussi de la créosote.